# DEMANDE DE BREVET EUROPEEN

(11) **EP 4 570 202 A2**
(43) Date de publication de la demande: **18.06.2025**
(21) Numéro de dépôt: 25167518.7
(22) Date de dépôt: 20.02.2018
(51) Int. Cl.: A61B 17/88

(54) **VIS POUR OSTÉOSYNTHÈSE COMPORTANT UN INDEXAGE ANGULAIRE PAR RAPPORT AU TOURNEVIS**

(30) Priorité: 22.02.2017 FR 1751390
(62) Demande divisionnaire de: 18710093.8
(71) Demandeur: Novastep, 35000 Rennes (FR)
(72) Inventeur: GIROD, Loïc, 35580 GOVEN (FR); LE BESQUE, Rémi, 35170 BRUZ (FR)
(74) Mandataire: Germain Maureau

(57) **Abrégé**

L'invention a pour objet une vis de serrage pour ostéosynthèse comportant un filetage (22) disposé suivant un axe principal (A), et à son extrémité arrière une empreinte présentant autour de l'axe principal (A) des formes régulières réparties angulairement, adaptées pour recevoir des formes complémentaires d'un tournevis s'engageant dessus, la face arrière de la vis (28) présentant une inclinaison par rapport à un plan transversal de la vis (20), cette vis étant remarquable en ce que l'empreinte de la vis comporte au moins une forme particulière différente des autres formes régulières.

## Description

La présente invention concerne une vis de serrage prévue pour être actionnée par un tournevis, et un tournevis adapté pour cette vis de serrage. En particulier l'invention peut s'appliquer au domaine de la chirurgie de réparation osseuse, notamment pour serrer et fixer une vis d'ostéosynthèse dans un fragment d'os à stabiliser. Plus particulièrement l'invention peut s'appliquer à la chirurgie des extrémités des membres, comme les pieds, les chevilles, les mains ou les poignets.

L'ostéosynthèse est une opération chirurgicale qui consiste à fixer entre eux deux parties osseuses comme les fragments d'un os coupé ou cassé, ou deux os proches l'un de l'autre, grâce à des éléments axiaux comme des vis ou des broches réalisées dans des matériaux métalliques tolérés par l'organisme, présentant une forme adaptée aux éléments à traiter.

En particulier l'ostéosynthèse peut être appliquée pour des opérations d'ostéotomie du type chevron, en approche mini-invasive.

L'hallux-valgus est une déformation fréquente de l'avant-pied qui se traduit par une déviation en dedans appelée varus du premier métatarsien, et une déviation en dehors appelée valgus de la première phalange. Un type d'intervention chirurgicale permettant de corriger cette déformation comporte une ostéotomie en chevron, qui est fixée ensuite par une ou plusieurs vis d'ostéosynthèse en approche mini-invasive.

L'approche chirurgicale mini-invasive consiste à réaliser de petites incisions dans la peau et les tissus pour passer les instruments et les implants nécessaires à la correction. On minimise ainsi la taille des cicatrices, ce qui offre une réhabilitation plus rapide des tissus par rapport à une approche classique dite à ciel ouvert.

Les vis utilisées pour l'ostéosynthèse comportent une longueur totalement ou partiellement filetée, et une tête arrière comprenant en bout une empreinte creuse prévue pour être actionnée par un tournevis. En particulier les têtes peuvent présenter une face d'extrémité arrière biseautée, inclinée généralement à 45° par rapport à l'axe principal de la vis, qui doit être disposée suivant une orientation particulière afin d'épouser le contour externe de l'os après l'introduction complète de cette vis à l'intérieur, pour limiter l'endommagement des tissus environnants.

Pour une intervention chirurgicale mini-invasive avec ce type de vis, à l'approche de la phase finale de serrage de la vis dans le site osseux, les tissus peuvent cacher visuellement la face arrière biseautée de la vis.

Par ailleurs un type d'empreinte creuse connu pour une vis d'ostéosynthèse, présenté notamment par le document WO-A1-2015185828, comporte six lobes répartis circonférentiellement autour de l'axe, recevant une forme mâle complémentaire formée au bout du tournevis.

La forme globalement cylindrique à six lobes de la vis ou du tournevis présente de plus sur certains lobes une partie légèrement conique permettant par une poussée axiale un petit serrage du tournevis dans l'empreinte, afin d'assurer un maintien provisoire de la vis au bout du tournevis. De cette manière on peut éviter une perte de la liaison du tournevis avec la vis avant ou pendant le montage.

De plus pour une vis présentant une face arrière biseautée, il est connu d'utiliser un tournevis présentant un repère visuel indiquant une orientation autour de son axe, ce qui permet en fixant la vis sur le tournevis de manière particulière par rapport à ce repère, de connaître constamment en fin de vissage la position de cette face arrière inclinée alors qu'elle n'est plus visible.

Toutefois si le vissage n'est pas achevé alors que la face arrière de la vis cachée par les tissus n'est plus apparente, en cas de désaccouplement du tournevis sur l'empreinte de la vis, par exemple lors d'une fausse manœuvre, le chirurgien doit réaliser une opération complémentaire pour retrouver l'inclinaison de cette face arrière, comme le dévissage de la vis, ou la prise d'un cliché aux rayons X visualisant cette inclinaison. On a alors une perte de temps pendant l'opération du patient qui est dommageable.

La présente invention a notamment pour but d'éviter ces inconvénients de la technique antérieure.

Elle propose à cet effet une vis de serrage pour ostéosynthèse comportant un filetage disposé suivant un axe principal, et son extrémité arrière une empreinte présentant autour de l'axe principal des formes régulières réparties angulairement, adaptées pour recevoir des formes complémentaires d'un tournevis, la face arrière de la vis présentant une inclinaison par rapport à un plan transversal de la vis, cette vis étant remarquable en ce que son empreinte comporte au moins une forme particulière différente des autres formes régulières.

Un avantage de cette vis de serrage est qu'en réalisant sur l'extrémité avant du tournevis des formes complémentaires de celles de la vis, comprenant une forme particulière complémentaire, on obtient une orientation unique du tournevis engagé sur l'empreinte de la vis.

En cas de désaccouplement du tournevis avant la fin d'opération de serrage alors que la face arrière de la vis n'est plus visible, le chirurgien fait tourner doucement le tournevis en exerçant une légère pression jusqu'à son emboitement sur l'empreinte de cette vis, permettant ainsi de retrouver l'orientation unique imposée par la forme particulière.

Le chirurgien termine alors son serrage en surveillant un repère visuel d'orientation du tournevis, pour connaître avec certitude l'orientation de la face arrière inclinée de la vis.

La vis de serrage pour ostéosynthèse selon l'invention peut comporter de plus une ou plusieurs des caractéristiques suivantes, qui peuvent être combinées entre elles.

Avantageusement, la vis de serrage comporte cinq formes régulières. On s'approche ainsi des empreintes de vis présentant généralement six formes régulières.

Avantageusement, la forme particulière couvre autour de l'axe un secteur angulaire équivalent à celui d'une forme régulière. Ce type d'empreinte peut être facilement dérivé d'une empreinte connue comportant six formes régulières.

Selon un mode de réalisation, les formes régulières constituent des lobes disposés sur une couronne centrée sur l'axe, comprise entre un cercle intérieur de petit rayon et un cercle extérieur de grand rayon. Ce type de forme permet de transmettre un couple élevé.

Selon un autre mode de réalisation, les formes régulières comportent un premier arc de cercle centré sur l'axe, puis un deuxième arc de cercle dépassant vers l'extérieur par rapport à ce premier arc de cercle.

Avantageusement, la forme particulière présente un rayon constant qui est centré sur l'axe.

Dans ce cas, le rayon constant de la forme particulière peut être le petit rayon du cercle intérieur des formes régulières constituant des lobes.

Avantageusement, l'empreinte comporte une forme globalement cylindrique disposée parallèlement à l'axe, comprenant une partie légèrement conique. Cette partie conique permet un blocage sur le tournevis.

Selon un mode de réalisation, le filetage de la vis s'étend sur toute la longueur de cette vis, et présente un diamètre plus important du côté arrière de la vis.

Selon un autre mode de réalisation, la vis comporte un filetage avant s'étendant sur une partie avant de la longueur de la vis, et un filetage de tête disposé à l'arrière de cette vis, séparé du filetage avant, présentant un diamètre plus important que ce filetage avant.

L'invention a aussi pour objet un tournevis prévu pour serrer des vis présentant l'une quelconque des caractéristiques précédentes, comportant à l'avant une pointe présentant des formes complémentaires à celles de l'empreinte des vis permettant de s'engager dessus.

Avantageusement, le tournevis comporte un repère angulaire visuel.

Avantageusement, la pointe du tournevis comporte une forme globalement cylindrique disposée parallèlement à l'axe, comprenant une partie légèrement conique. Cette partie conique permet un blocage de la vis sur le tournevis.

D'autres caractéristiques et avantages de l'invention ressortiront à la lecture de la description qui suit, donnée uniquement à titre d'exemple, en référence aux figures annexées suivantes :
- les figures 1a, 1b et 1c présentent successivement sur un pied en vue de dessus, trois étapes d'une chirurgie pour réduire un hallux valgus du gros orteil ;
- la figure 2 est une vue de côté de ce pied après l'intervention ;
- la figure 3 présente une vis selon l'invention utilisée pour cette intervention ;
- la figure 4 est une vue extérieure du pied après l'insertion de la vis ;
- la figure 5 est une vue complète des os du pied présentant l'insertion de la vis sur le métatarse ;
- la figure 6 est une vue arrière de la vis présentant son empreinte d'entraînement ;
- la figure 7 présente un tournevis prévu pour cette vis ; et
- la figure 8 présente une vis selon une variante.

Pour plus de clarté, les éléments identiques ou similaires sont repérés par des signes de référence identiques sur l'ensemble des figures.

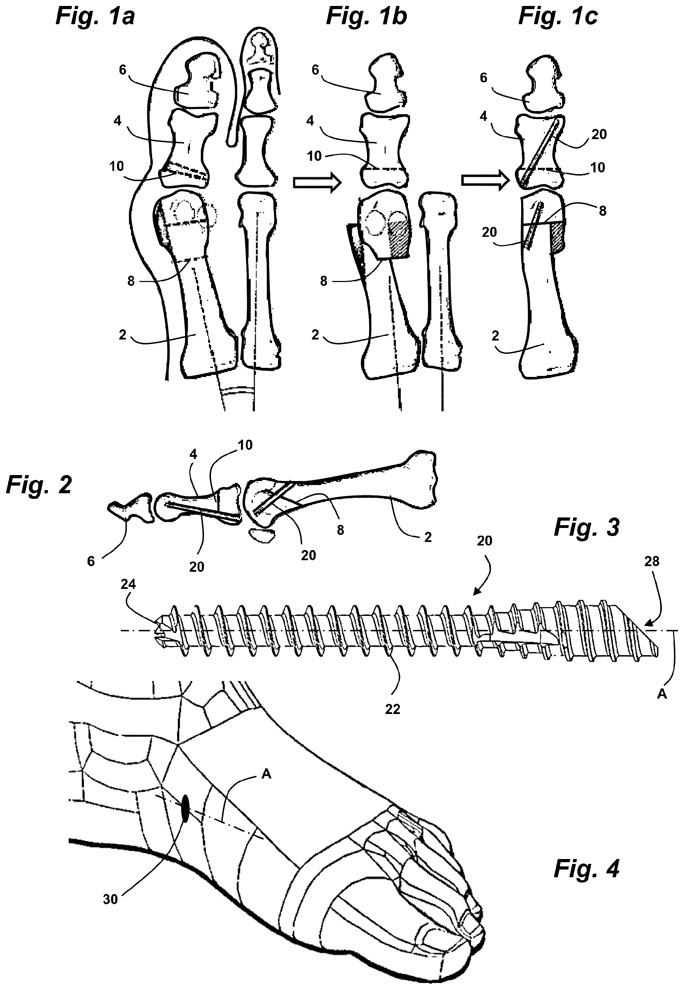

La figure 1a présente un pied comportant successivement vers l'avant pour le gros orteil, un métatarse 2, une phalange proximale 4 et une phalange distale 6.

Le côté intérieur du pied est celui tourné vers l'axe du corps. Le métatarse 2 présente une inclinaison anormale vers l'intérieur, et la phalange proximale 4 une inclinaison anormale vers l'extérieur.

La première étape de l'opération chirurgicale prévoit une coupe 8 du métatarse 2 dans sa partie avant, formée en biais, et une double coupe 10 de la phalange proximale 4 dans sa partie arrière afin de retirer une partie de l'os formant un coin.

La figure 1b présente le déplacement de la partie avant du métatarse 2 vers l'extérieur par rapport à la partie arrière, et un redressement de la phalange proximale 4 vers l'intérieur en ayant retiré la partie de l'os formant le coin. On obtient sensiblement un alignement suivant l'axe du pied, du métatarse 2 et de la phalange proximale 4 ainsi modifiés.

La figure 1c et la figure 2 présentent l'insertion d'une première vis de serrage 20 dans les deux parties du métatarse 2 et d'une deuxième vis dans les deux parties de la phalange proximale 4, pour fixer à chaque fois ces deux parties ensemble.

Chaque vis de serrage 20 entre sur le côté intérieur du pied en venant de l'arrière, après avoir pratiqué une incision minimum sur les tissus disposés dans l'axe A de la vis.

La figure 3 présente une vis 20 comportant un filetage 22 sur toute sa longueur, comprenant un diamètre qui augmente progressivement dans une partie arrière. L'extrémité avant de la vis 20 se termine par une forme 24 assurant la formation du filetage dans les parties d'os.

La face d'extrémité arrière plane 28 de la vis 20 présente une inclinaison par rapport à un plan transversal de la vis, formant un biseau qui peut être de 45°. Cette face d'extrémité 28 comporte une empreinte creuse dirigée axialement, centrée sur l'axe de la vis A, prévue pour recevoir une forme complémentaire d'un tournevis afin de l'entraîner en rotation.

En variante la vis peut-être une vis de compression comportant un filetage avant s'étendant sur une partie avant de sa longueur, puis une partie lisse, et enfin un filetage de tête disposé à l'arrière de cette vis, présentant un diamètre plus important que le filetage avant.

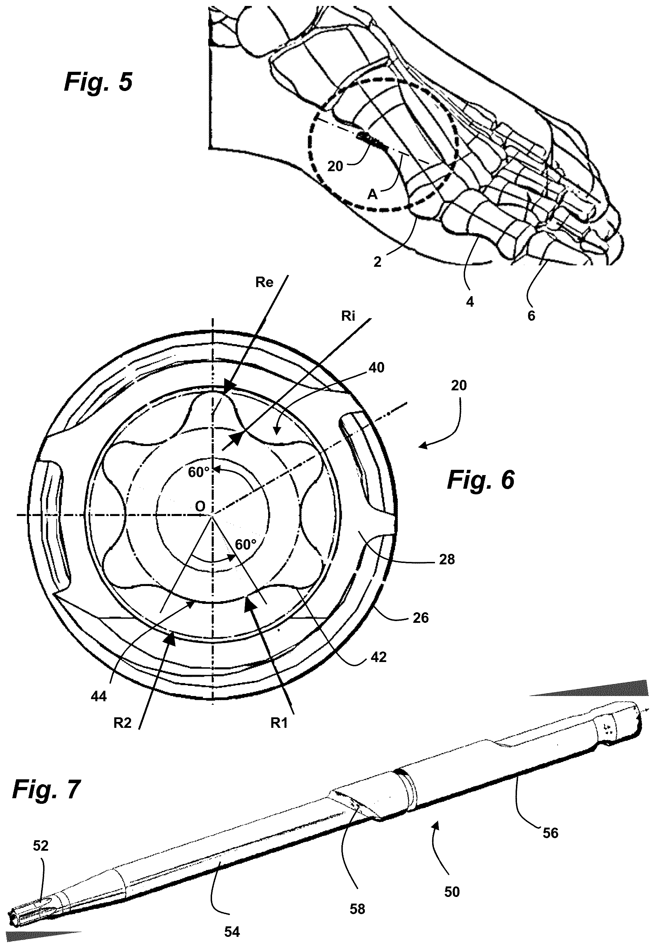

Les figures 4 et 5 présentent une petite incision 30 formée sur le côté intérieur du pied, permettant d'engager les outils pour couper les tissus et d'insérer une vis 20 sur le métatarse 2. D'autres incisions permettent de couper les os 2, 4, et d'insérer la deuxième vis 20. De cette manière on réalise un minimum de traumatisme sur les tissus.

A la fin du serrage des vis 20 on ajuste la position angulaire de chaque vis pour aligner sensiblement sa face arrière biseautée 28 sur la surface extérieure de l'os, afin d'assurer un minimum de contraintes ou de traumatismes sur les tissus environnants.

La figure 6 présente l'empreinte en creux 40 formée sur la face arrière 28 de la vis 20, constituant un cylindre parallèle à l'axe de la vis A, comprenant une succession de cinq formes identiques qui sont des lobes réguliers 42 disposés sur une couronne centrée sur l'axe, comprise entre un cercle intérieur de petit rayon R1 et un cercle extérieur de grand rayon R2.

Chacun des cinq lobes 42 du creux de l'empreinte 40 disposé suivant un angle de 60° par rapport au centre O, comporte successivement un rayon concave Re puis un rayon convexe Ri. Un angle de 60° comporte une forme particulière en arc de cercle 44, comprenant une absence de lobe, qui suit le cercle intérieur de petit rayon R1.

L'empreinte 40 en forme d'étoile peut reproduire en particulier une empreinte connue sous l'appellation commerciale « Torx », avec un lobe en moins.

La figure 7 présente un tournevis 50 comportant à l'avant d'une tige cylindrique métallique 54 une pointe 52 constituant un cylindre centré sur l'axe, prévu pour s'ajuster avec un jeu minimum dans l'empreinte 40 de la face arrière 28 de la vis 20.

En particulier la pointe avant 52 comporte une succession de cinq formes constituant des lobes réguliers, s'étendant chacun suivant un angle de 60°. Sur les 60° restant on a une partie circulaire formée par une absence de lobe, qui suit le cercle intérieur de rayon du R1.

On obtient un positionnement unique de la vis 20 sur le tournevis 50, qui est donné par l'ajustement l'un sur l'autre des deux parties circulaires. Pour le montage initial de la vis 20 sur le tournevis 50, ou après un début d'insertion si le chirurgien déboîte le tournevis de la vis et veux le remettre ensuite, on obtient forcément le positionnement angulaire unique.

En particulier l'empreinte 40 de la vis 20 ou la pointe avant 52 du tournevis 50, peut comporter sur certains lobes une forme légèrement conique s'ouvrant vers l'arrière pour la vis ou se refermant vers l'avant pour le tournevis, afin d'assurer après une poussée axiale un maintien de la vis sur le tournevis, comme présenté par le document de l'art antérieur cité ci-dessus.

Le tournevis 50 comporte vers l'arrière une zone de connexion pour un manche 56, présentant une face avant 58 formant un biseau incliné à 45°, disposée parallèlement à la face arrière biseautée 28 de la vis 20 quand elle est fixée sur ce tournevis. De cette manière le chirurgien visualise directement en regardant le manche la position angulaire de la face arrière biseautée 28 de la vis 20.

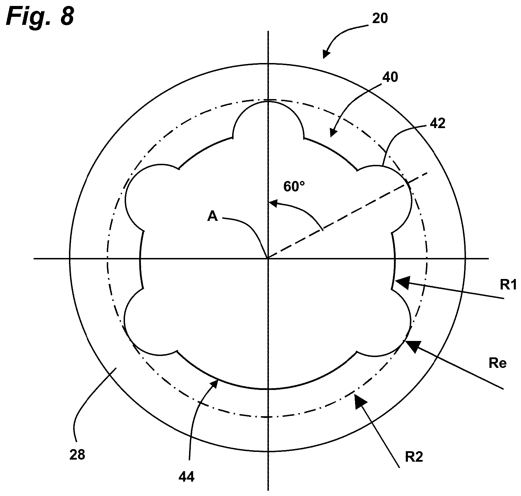

La figure 8 présente en variante une empreinte 40 formée sur la face arrière 28 de la vis 20, constituant un cylindre parallèle à l'axe de la vis A, comprenant une succession de cinq formes régulières identiques couvrant chacune un angle de 60°, comprises entre les cercles centrés sur l'axe A, de petit rayon R1 et de grand rayon R2.

Chaque forme régulière comporte un arc de cercle qui suit le petit rayon R1, puis un lobe 42 formé par un arc de cercle de rayon convexe Re- tourné vers l'extérieur, qui va jusqu'au cercle extérieur de grand rayon R2.

Un des lobes est absent laissant à la place sur un angle de 60° une forme particulière en arc de cercle 44 qui suit le cercle intérieur de petit rayon R1.

Un procédé de fabrication de l'empreinte 40 de la vis 20, simple à mettre en œuvre, comporte une succession de cinq perçages pour former les lobes 42, puis un fraisage centré sur l'axe suivant le petit rayon R1, pour terminer la forme cylindrique de cette empreinte.

La vis 20 suivant l'invention ainsi que le tournevis spécifique 50 assurent au chirurgien une facilité, une sécurité et une vitesse d'exécution des opérations chirurgicales utilisant ce type de vis.

En variante l'empreinte 40 de la vis 20 peut présenter d'autres formes. On peut utiliser en particulier des formes normalisées, comme une forme hexagonale qui est plus facile à réaliser, mais présente une capacité de transmission de couple réduite.

## Revendications

1. Vis de serrage pour ostéosynthèse comportant un filetage (22) disposé suivant un axe principal (A), et à son extrémité arrière une empreinte (40) présentant autour de l'axe principal (A) des formes régulières (42) réparties angulairement, adaptées pour recevoir des formes complémentaires d'un tournevis (50) s'engageant dessus, la face arrière de la vis (28) présentant une inclinaison par rapport à un plan transversal de la vis (20), **caractérisée en ce que** l'empreinte de la vis (40) comporte au moins une forme particulière (44) différente des autres formes régulières (42).

2. Vis de serrage selon la revendication 1, **caractérisée en ce qu'**elle comporte cinq formes régulières (42).

3. Vis de serrage selon la revendication 1 ou 2, **caractérisée en ce que** la forme particulière (44) couvre autour de l'axe (A) un secteur angulaire équivalent à celui d'une forme régulière (42).

4. Vis de serrage selon l'une quelconque des revendications précédentes, **caractérisée en ce que** les formes régulières (42) constituent des lobes disposés sur une couronne centrée sur l'axe (A), comprise entre un cercle intérieur de petit rayon (R1) et un cercle extérieur de grand rayon (R2).

5. Vis de serrage selon l'une quelconque des revendications 1 à 3, **caractérisée en ce que** les formes régulières (42) comportent un premier arc de cercle centré sur l'axe (A), puis un deuxième arc de cercle (Re) dépassant vers l'extérieur par rapport à ce premier arc de cercle.

6. Vis de serrage selon l'une quelconque des revendications précédentes, **caractérisée en ce que** la forme particulière (44) présente un rayon constant (R1) qui est centré sur l'axe (A).

7. Vis de serrage selon l'une quelconque des revendications précédentes, **caractérisée en ce que** l'empreinte (40) comporte une forme globalement cylindrique disposée parallèlement à l'axe (A), comprenant une partie légèrement conique.

8. Vis de serrage selon l'une quelconque des revendications précédentes, **caractérisée en ce que** son filetage (22) s'étend sur toute la longueur de la vis (20), et présente un diamètre plus important du côté arrière de cette vis (20).

9. Vis de serrage selon l'une quelconque des revendications 1 à 7, **caractérisée en ce qu'**elle comporte un filetage avant s'étendant sur une partie avant de la longueur de la vis (20), et un filetage de tête disposé à l'arrière de cette vis (20), séparé du filetage avant, présentant un diamètre plus important que ce filetage avant.

10. Tournevis prévu pour serrer des vis selon l'une quelconque des revendications précédentes, comportant à l'avant une pointe (52) présentant des formes complémentaires à celles de l'empreinte (40) permettant de s'engager dessus, comportant un repère angulaire visuel (58), **caractérisé en ce que** sa pointe (52) comporte une forme globalement cylindrique disposée parallèlement à l'axe, comprenant une partie légèrement conique.
